# EUROPEAN PATENT APPLICATION

(11) **EP 2 361 502 A1**
(43) Date of publication of application: **31.08.2011**
(21) Application number: 10001221.0
(22) Date of filing: 05.02.2010
(51) Int. Cl.: A01N 25/28, A01N 25/34, A61K 9/51, A61K 9/127, A61K 9/133, A61L 15/22, A01N 59/00, A01N 59/04, A01N 59/12, A01N 47/44, A01N 43/90, A01N 43/54, A01N 33/12

(54) **Active antimicrobial compositions, coatings and methods for controlling pathogenic bacteria**

(71) Applicant: Max-Planck-Gesellschaft zur Förderung der Wissenschaften e.V., 80539 München (DE); University Of Bath, Bath BA2 7AY (GB)
(72) Inventor: Förch, Renate, 55127 Mainz (DE); Jenkins, Andrew, Bath BA2 2TB (GB)
(74) Representative: Katzameyer, Michael

(57) **Abstract**

The invention relates to novel active antimicrobial compositions, coatings, in particular for wound dressings, and methods for controlling pathogenic bacteria. The present invention uses the fact that pathogenic bacteria secrete cell membrane-lysing and/or cell membrane permeating agents for a responsive system wherein the action of antimicrobial agents is mediated by the presence of pathogenic bacteria itself.

According to the present invention biomimetic antimicrobial nanocapsules are provided which have at least one antimicrobial agent encapsulated in a membrane, wherein the nanocapsules are capable to release the antimicrobial agent(s) in response to the presence of any pathogenic bacteria which produce membrane-permeating or membrane-lysing agents. Preferably, the antimicrobial composition of the invention further comprises signalling nanocapsules having at least one type of signalling agent encapsulated in a membrane, and/or wound healing nanocapsules having at least one wound healing agent encapsulated in a membrane, which nanocapsules are also capable to release the respective agent(s) in response to the presence of pathogenic bacteria which produce membrane-permeating or membrane-lysing agents.

## Description

### Background of the Invention

The re-emergence of bacterial infection as a clinical problem in the late 20^{th} century, after it was reportedly beaten by the widespread use of antibiotics from the 1950's, has initiated a growing interest in developing systems that either kill bacteria or prevent bacterial attachment and growth. Many bacteria, e.g. *Pseudomonas aeruginosa,* have a strong tendency to attach to surfaces, colonize and form complex colonies, biofilms, and that has led to the development of antimicrobial coatings containing agents such as silver or quaternary ammonium cations (QACs) for control of bacterial growth. Such systems, while functional are essentially passive - they do not respond to their local environment and operate whether bacteria are present or not.

Nanocarriers can be used for delivering pharmaceutical drugs and antimicrobial agents. Such nanocarriers can be made sensitive to external stimuli such pH, temperature and chemicals (Drummond et al., Journal of Pharmaceutical Sciences Vol. 97, No. 11 (2008); Kiparissides and Kammona, Phys. Stat. Sol. (c) 5, No. 12, 3828-3833 (2008)). In a recent paper, (J. Am. Chem. Soc. (2009), 131, 2469-2471) Griset et al. described the synthesis and testing of pH sensitive polymeric nanoparticles which respond to their local environment. At pH 7.4, the hydrogel nanoparticles were stable, at pH 5, the polymer hydrolysed and the particle swelled to up to six times its original diameter and in doing so released an encapsulated drug, paclitaxel, used as a chemotherapeutic agent against lung carcinomas. Mi et. al (J. Biomedical Mat. Res. 2002, 59, 438-449) have investigated a chitosan bilayer in a wound dressing that was reported to give a burst response, followed by slow release of the topical antimicrobial silver sulfadiazine. This system has the potential advantage of combining the reported wound healing properties of chitin/chitosan with antibiotic action.

However, the systems of the prior art still suffer from various drawbacks. For example, they operate whether pathogenic bacteria are present or not, resulting in premature and excessive consumption of the active agent, their efficacy is often insufficient, and/or it is often difficult or laborious to assess whether the pathogenic bacteria or the active agent is/are (still) present.

Consequently, the object underlying the present invention is to provide improved antimicrobial compositions, in particular antimicrobial coatings and wound dressings, as well as improved methods for controlling pathogenic bacteria, which overcome the above mentioned drawbacks of the prior art.

This object has been achieved by providing the novel antimicrobial nanocapsules, compositions and wound dressings according to claims 1-11, as well as by providing the methods of claims 12-16.

### Description of the Invention

The present invention is based on the consideration that pathogenic bacteria differ from non-pathogenic bacteria in important respects and that these differences can be used to develop an active antimicrobial system which responds to the presence of such pathogenic bacteria.

The strict definition of pathogenic bacteria compared with non-pathogenic bacteria may be sometimes ambiguous, since it depends on the location of bacteria on or in a person and the behaviour in a specific environment. However, for the purposes herein, pathogenic bacteria can be clearly defined as those bacteria which secrete virulence factors such as toxins that cause tissue damage. Pathogenic bacteria cause harm to their eukaryotic hosts by secreting enzymes and toxins that damage cells and connective matrix material of the tissues. Many of these toxins and enzymes act as cell membrane-lysing and/or cell membrane-permeating agents.

One of the most important bacteria in nosocomial infections *- Staphylococcus aureus -* displays a large armoury of specific proteins that enable infection, so called virulence factors. They include:
(i) Adhesins that allow it to attach to tissues,
(ii) Enzymes such as hyaluronidase or lipases that degrade tissue matrix proteins and allow deep tissue penetration.
(iii)Toxins, such as α-haemolysin, which can permeate the cell membrane.
(iv) Other virulence factors such as leukocidin, leukotoxin and staphylysin act as cytotoxins, directly killing host cells allowing further tissue invasion and suppressing their own immune destruction.
(v) Proteins such as coagulase and Protein A further act to suppress the host immune system.

The secretion of such virulence factors to alter the properties of the host environment is common to all pathogenic bacteria. Knowledge of the enzymes and toxins secreted by specific pathogens and their mode of attack on eukaryotic cells provides a unique opportunity for detecting the presence of pathogens and using this to combat infection.

The present invention uses the fact that pathogenic bacteria secrete cell membrane-lysing and/or cell membrane permeating agents for a responsive system wherein the action of antimicrobial agents is mediated by the presence of pathogenic bacteria itself. According to the present invention biomimetic antimicrobial nanocapsules are provided which have at least one anti-microbial agent encapsulated in a membrane, wherein the nanocapsules are capable to release the antimicrobial agent(s) in response to the presence of any pathogenic bacteria which produce membrane-permeating or membrane-lysing agents. In the presence of non-pathogenic bacteria, which do not produce membrane-permeating or membrane-lysing agents, no release of the antimicrobial agent(s) takes place.

More specifically, the pathogenic bacteria may be selected from the group comprising (but not limited to) *Salmonellae* species, *Shigellae* species, pathogenic Escherichia coli such as *E. coli* 0157, *Staphylococcus,* in particular S. *aureus, Streptococcus, in particular* Group A and Group B *Streptococcus, e.g.* S. *pyogenes, S. agalactiae, Klebsiella* species, in particular *K. pneumoniae, Pseudomonas* species, in particular *P. aeruginosa, Lactobacillus, Helicobacter, Camphylobacter, Legionella, Listeria, Borellia, Yersinia, Bacillus* and *Vibrio.*

The only qualifying requirement for pathogenic bacteria is that the bacteria can either directly damage cells by means of their virulence factors and/or stimulate an immune response that is also damaging to host cells/tissue. An example being toxic-shock toxin, a secretion factor of some *S. aureus* bacteria which can cause death in young children as a consequence of the resultant cytokine cascade and immune over response.

The membrane-permeating or membrane-lysing agents may be any toxic agents produced by pathogenic bacteria which damage the cell walls of healthy cells - or induce such damage indirectly. Typically, they are selected from the group of virulence factors, in particular lipases, phospholipases including phospholipase A2, toxic shock toxin (TSST-1), hyaluronidases, pore-forming toxins, in particular haemolysin, S. *aureus* a, β and δ toxins, streptolysin and leukocidin.

The natural role of enzymes such as hyaluronidases or lipases secreted by pathogenic bacteria is the degradation of tissue via enzymatic cleavage of tissue matrix proteins. Each of these types of enzymes attacks selectively a particular type of bond. Lipases, for instance, are known to attack and hydrolyse acyl bonds. This degradative behaviour has been exploited for the controlled release of hydrophobic drugs from polymeric carriers, such as nanoparticles, micelles or vesicles (M. Flasza-Baron et al. (2007), Regen. Medicine 2, 903-918). For instance, the lipases from *Thermomyces lanuginosus* or *Pseudomonas aeruginosa* were reported to hydrolyse man-made and natural biodegradable polymers. Typical examples are polymers based on lactide, glycolide, ε-caprolactone, trimethylene carbonate, or natural polymer, such as cellulose or polyhydroxyalkanoates (Whitaker et al., Burns (2007) 33, 1015-1020).

The family of hyaluronidases comprise of both hydrolases and lyases. The latter ones are found only in bacteria and are hence one sub-family of target enzymes for the synthesis of materials for nanocapsules with designed 'weak' bonds which are susceptible to cleavage by bacterium-specific enzymes. Since only five hydrolases (the so-called chondroitinases) are known in humans, bacterium-specific hydrolases constitute a further class of suitable target enzymes.

The membrane of said nanocapsules may be of any material which can be lysed or permeated by the action of toxins secreted by pathogenic bacteria, e.g. by the lipases and hyaluronidases mentioned above. Thus, the term "membrane" as used herein includes any kind of biomimetic shell which resembles a cell membrane insofar as it can be lysed or permeated by the action of agents/toxins secreted by pathogenic bacteria.

Some non-limiting examples of suitable nanocapsules are phospholipid-fatty acid vesicles, with properties similar to naturally occurring vesicles; synthetic multi-component fatty acid/lipid vesicles, in particular comprising polymerized acetylene fatty acids, lipids and optionally other polymerized or polymerizable components such as for example tricosadiyanoic acid, dimyristoylcholine (DMPC), dimyristoylserine (DMPS), and cholesterol (Reppy and Pindzola, Chem. Commun. 2007, 4317-4338; Kolusheva et al., J. Lipids Research, 2003, 44, 65-71); amphiphilic block copolymer nanocapsules (e.g. prepared as described by Discher and Eisenberg, Science 2002, 297, 967-973), Soo and Eisenberg, J. Polym. Sci. Part B: Polym. Phys. 2004, 42, 923-938); nanocapsules comprising natural or synthetic polymers, e.g. based on lactide, glycolide, ε-caprolactone, trimethylene carbonate, cellulose, or polyhydroxyalkanoates; protein capsules; or other containment systems comprising of self-assembled and/or polymerized organic molecules.

In a specific embodiment, nanocapsules may be prepared from nanodroplets using the so-called mini-emulsion process as described by K. Landfester in Annual Reviews of Material Research, 2006, 36, 231-279. This process can be performed with different surfactants and capsule sizes between 30 nm and 500 nm can be obtained with a narrow size distribution.

The size of the nanocapsules used in the present invention may vary in a broad range, depending on the specific conditions of application. Typically, the size will be in the range from 10 to 10000 nm, preferably 20 to 800 nm, more preferably 50 to 400 nm.

An antimicrobial composition of the present invention comprises at least antimicrobial nanocapsules as described above having incorporated one or more antimicrobial agent(s) and may also comprise further additives used in the prior art for antimicrobial compositions. The antimicrobial agent(s) may be any agent used for controlling of pathogenic agent and will be selected in dependency from the specific application of the antimicrobial composition (e.g. cleaning composition, wound dressing etc.) and the specific pathogenic bacteria to be controlled. Specifically, the antimicrobial agent may be selected from the group of any water soluble antimicrobial and/or antibiotic agent including (but not limited to) flucoxacillin, chloroxacillin, silver sulfadiazine, sodium azide, chlorhexidine, sodium bicarbonate, povidone-iodine, quaternary ammonium cations (QACs).

The amount of antimicrobial agent contained in said nanocapsules is not critical and will depend on the specific application of said nanocapsules and the specific antimicrobial agent used. Typically, the nanocapsules will contain the antimicrobial agent in an amount in the range from 1 ng to 1000 pg/capsule, more specifically in an amount of from 10 ng to 100 pg/capsule.

In a preferred embodiment, the antimicrobial composition of the invention comprises several types of nanocapsules and/or hybrid nanocapsules containing different types of active agents in the same or different compartments.

Hybrid nanocapsules can for example be obtained by combining polymeric nanocapsules, such as mini-emulsion-based polymeric nanocapsules or block copolymer nanocapsules, and fatty acid/(phospho)lipid vesicles or synthetic multi-component lipid vesicles, such as lipid vesicles comprising polymerized acetylene fatty acids and/or other polymerized or polymerizable components (e.g. as indicated above). Preferably the polymeric nanocapsules will be used as inner capsules which are enclosed in larger fatty acid/(phospho)lipid vesicles or synthetic multi-component fatty acid/lipid vesicles.

Typically, the diameters of the inner nanocapsules will be less than 100 nm, whereas the outer capsules will have diameters larger than 100 nm.

It may be additionally advantageous to functionalize the nanocapsules and in particular the above hybrid nanocapsules on the outer surface, so that they can undergo hydrolysis or condensation reactions with functionalized surfaces and/or to conjugate the outer surface with PEG (e.g. for stabilization or prevention of unspecific enzymatic attack).

The signalling agent(s) may be any signalling agent(s) known in the art for similar purposes. More specifically, the signalling agent may be selected from group comprising organic and inorganic dyes such as (for example) pH sensitive dyes such as Crystal violet, bromothymol blue, methyl orange, pH and concentration sensitive fluorometric dyes such as carboxyfluorescein.

The wound healing agent may be any agent which supports tissue repair and tissue growth and/or which suppresses the formation of scars. Some non-limiting examples are growth factors including epidermal growth factors (EgF) and insulin like growth factors (IgFs). In addition to assist healing, protease inhibitors such as secretory leukocyte protease inhibitor (SLPI), hyaluronic acid, and serine protease inhibitors may be included.

In a preferred embodiment of said antimicrobial composition, the signalling nanocapsules and/or wound healing nanocapsules (which may be present as separate nanocapsules or as a component of hybrid nanocapsules) are also capable to release the respective agent(s) in response to the presence of pathogenic bacteria which produce membrane-permeating or membrane-lysing agents. However, it is also possible to use signalling nanocapsules or wound healing capsules which can be induced to release their content by other external stimuli, such as light, temperature, pH etc. Such an embodiment may be advantageous in some cases.

The release of the different agent(s) may be adjusted to take place simultaneously or at different times. Preferably, in the antimicrobial composition of the invention the signalling nanocapsules and/or wound healing nanocapsules are adapted to release the respective agent(s) after a predetermined time period after release of the antimicrobial agent(s). Such a delayed release of signalling agent(s) provides considerable advantages. For example, it is possible to adjust the release the signalling agent(s) so that it takes places only after depletion of the antimicrobial agent(s). Thus, the release of signalling agent indicates that a fresh antimicrobial composition, e. a fresh wound dressing has to be applied. However, a simultaneous release of signalling agent and antimicrobial agent may also be advantageous, since, e.g. the presence and/or degree of an infection by a specific pathogen can be indicated. In further specific embodiments, different signalling nanocapsules with different release characteristics (for example one type of nanocapsules providing delayed release and another type providing immediate release of the respective agent) and/or different signalling agents may be used.

The release behaviour of nanocapsules can be tailored by modifying the characteristics, e.g. the thickness and/or the degree of cross-linking, of the membrane-forming material. Techniques for modifying the release charac-teristics of nanocapsules are known from the prior art, e.g. as described by Antipov et al. in J. Phys. Chem. B, 2001, 105 (12), 2281-2284, by R. Jalil and J. R. Nixon in Journal of Microencapsulation, 1990, Vol. 7, No. 3, 297-325, by K. Avgoustakis, Current Drug Delivery, Vol. 1, No. 4, October 2004, 321-333(13), by Bingbing Jiang et al. in Acta Biomaterialia, Vol. 2, Issue 1, January 2006, 9-18, by Germain et al. in Advanced Materials, Vol. 18, Issue 21, 2868-2871, 2006, by Bin Mu et al. in Nanoscale Research Letters, Vol. 4, No. 7, July 2009, 773-777(5).

In a preferred embodiment of the invention, hybrid nanocapsules as described above are used to control the release kinetics of different active agents. The different agents, e.g. an antimicrobial agent and a signalling agent, will be separated in different compartments, e.g. formed by different shells or spheres. For example, according to one embodiment the outer sphere consists of a fatty acid/lipid bilayer which surrounds and includes at least one inner sphere, which is the shell or membrane of a smaller nanocapsule, e.g. a mini-emulsion based nanocapsule or other polymeric nanocapsule or a protein capsule or lipid vesicle, and antimicrobial agent(s) encased in the space between the outer sphere and the inner sphere. The inner sphere contains the signalling agent, such as dye molecules which may be detectable as such or "switched on" after release.

The resulting hybrid nanocapsules will be able to show a coupled (cascade-like) release behaviour of the dye and the antimicrobial components. This allows one to have different release kinetics of the two containers. The hybrid capsules will release the antimicrobial agent(s) upon initial attack of the membrane-lysing or membrane-permeating agent(s) (such as enzymes or virulence factors or toxins) of the pathogenic bacteria. Only if the concentration of the membrane-lysing or membrane-permeating agent(s) remains high (i.e. when the antimicrobial agent can no longer eliminate the threat of bacterial infection) the enclosed smaller nanocapsules containing the signalling agent(s) will also be lysed (e.g. enzymatically digested) or permeated and release the (e.g. colorimetric) indicator for infection. Thus, for example false or premature alarms at low bacterial concentrations can be avoided.

In a specific embodiment, the antimicrobial composition of the invention is provided on a substrate surface. The substrate surface may be any surface which permits the physical or chemical bonding of the nanocapsules to said surface. The physical bonding may be effected by e.g. electrostatic forces, van der Waal's interactions or other adhesive forces known in the art. Specifically, said substrate may be selected from the group of woven or non-woven fabrics, threads and yarns, solid polymeric surfaces, polymeric films, metal, glass or ceramic surfaces and/or contained within a hydrogel matrix such as chitosan. The surface may be for example the surface of a wound dressing, suture material, implant, a microchip device or a diagnostic device.

The nanoscapsules can be provided on the substrate surface by any application or immobilization method known in the art. Some principal methods are spraying/drying processes, sol-gel processes, spin coating, or plasma based processes.

In particular for immobilization of nanocapsules on the surface of woven and non-woven textiles the following approaches are feasible:
(i) Activation of individual non-woven fibres using plasma assisted surface modification or the deposition of "adhesive" thin films;
(ii) Spraying of droplets containing the nanocapsules onto the surface, in particular textile surface;
(iii) Gentle plasma assisted modification to bind nanocapsules to a fibre surface;
(iv) Attachment of the nanocapsules to the surface by electrostatic forces.

In a preferred embodiment of the invention, plasma-assisted processes are used for the immobilization of nanocapsules on woven and non-woven fabrics and polymeric surfaces.

The modification of surfaces using plasma assisted processes is well established and the literature over the past 15 years demonstrates it to be a powerful and versatile method to sterilize, etch, chemically modify or to deposit nanometer thin organic, inorganic or nano-composite films (see, e.g., An and Friedman, J. Biomed. Mater. Res., 1998, 43, 338-348).

The treatment of textiles in the form of yarns, non-wovens and wovens using plasma assisted processes is already state of the art (see, e.g. "Plasma Technologies for Textiles", Chandos Publishing 2007). The products being treated are mostly aimed for technical applications such as filters, composite adhesion and for the improvement of properties such as dyeability, wettability, adhesion, durability and washability are key factors that influence widespread application. Plasma treatment of non-woven fabrics has been explored by a number of researchers and industries.

A plasma assisted technology for immobilizing the nanocapsules of the invention is especially advantageous because of its high efficiency, high cost effectiveness at a minimal consumption of chemicals and thus a minimal production of waste products. For example, the currently used sol-gel process for immobilization of nanoparticles onto surfaces compares unfavorably with a plasma assisted method of immobilization.

The typical sol-gel process requires a huge volume of solvent containing the particles which are to be brought onto a fabric. This is typically done in a dipping process. For a non-woven fabric of industrially relevant size (ca 2.5 m wide web) this will require several baths of 1000's of litres of solvent with suspended nanoparticles. The mixture forms a film on the fabric and the solvent is evaporated off leaving behind a thin functional film containing the nanoparticles. This inherently causes irritation to personnel and is generally toxic for the environment. The process can take many hours for completion of a 0.5 µm thick film.

In comparison, a low pressure plasma process for surface activation of fabrics can be run using compressed air with a 2-3m wide web being treated at a typical rate of ca. 5m/second. The spraying of nanocapsules can be effected in minimal amounts of solvent (e.g. water) onto a moving web previously activated either by low pressure, or atmospheric pressure plasma. The surface chemistry of the web and the nanocapsules can be matched to achieve optimum stability and control.

While the costs and maintenance for a large low pressure plasma reactor system are high (initial cost ca 400,000 to 1.000.000€), they are only a fraction of the long term costs for the sol-gel process and other wet chemical dipping processes used today. In the case of plasma processing, no solvents are used and there is no damage to the environment or health risks to staff. It is a physical process using regenerative sources, i.e. generally air, oxygen, nitrogen. As such, an overall reduction in energy consumption of at least 20% for the manufacture of comparable wound dressings using presently used technologies may be anticipated. Because of the speed of treatment an increase in the production efficiency of »30% is expected.

The antimicrobial nanocapsules, compositions and coatings of the present invention are suitable and advantageous for a broad range of applications, for example in the fields of health care, in particular for wound dressings, suture material and implants, in the clothing industry, food packing industry, hygiene articles, cleaning industry etc.

A preferred embodiment of the invention is a wound dressing, including a wound dressing for burns, comprising the antimicrobial nanocapsules, compositions or coatings as outlined above.

The skin is the largest organ in the body and performs numerous vital functions, including primary protection against infection by acting as a physical barrier. When this barrier is damaged, pathogens have a direct route to infiltrate the body, possibly resulting in infection. Immediately following a thermal burn, the surface of the burn wound is free of micro-organisms. However, deep cutaneous structures that survive the initial burn injury (e.g. sweat glands, hair follicles) often contain staphylococci, which colonize the wound surface during the subsequent 48 hours. Over the following 5-7 days, other microbes, including gram-negative and gram-positive bacteria, colonize the wound. These potential pathogens typically come from the patients' gastrointestinal tract, upper respiratory tract, or the hospital environment, transferring through contact with health care workers. Quite often, fungal infections develop later, either after broad-spectrum antibiotics have been administered or after wound care has been delayed. Specific examples for very relevant species of pathogenic bacteria in burns infections are: *Staphylococcal aureus, Streptococcus pyogenes, Pseudomonas aeruginosa* and *Klebsiella pneumoniae.*

The current state of the art in burn wound dressings includes plain wound dressings and different 'biological' dressings (dressings impregnated with growth factors). These are generally based on a polyethylene or polypropylene non-woven base material that is surface modified in wet chemical dip coating processes. Other materials include viscose and polyurethanes or mixtures of these materials.

Alternatively to these plain wound dressings, allografts from human cadavers are applied after cleaning of the burn wound. If the burn is deeper (3^{rd} degree), skin cover and healing can only be achieved by the grafting of the patient's own skin. Other approaches involve the cultivating of human dermal fibroblasts set in a natural human collagen matrix, which mimics the structure of skin and is intended to assist the regeneration of sub epithelial cells (the dermis) such as ICX-SKN™ made by Intercytex. Recent developments of biologically derived wound dressings containing both a collagen scaffold and growth factors, such as Biobrane™ have helped reduce the need for grafting, especially in more superficial burn injuries. However, these dressing are not anti-infective and dressings that encourage cell growth can even assist microbial growth. On the other hand, dressings such as Acticoat™, which contain silver to suppress microbial growth are also partially cytotoxic and can reduce epithelialisation and tissue growth. This, in turn, may lead to a significant increase in hospitalisation time and health care costs. Silver containing dressings continually expose the wound to the antimicrobial agent which may increase the rate of bacteria evolving resistance. The wound dressing of the present invention represents a considerable improvement over the wound dressings of the prior art since it is a "smart" dressing which releases its active agents, in particular its antimicrobial agent(s) only in the presence of pathogenic bacteria. Moreover, in a preferred embodiment said wound dressing is bifunctional or multifunctional and provides not only effective means for controlling pathogenic bacteria but concomitantly also agents which indicate in a very fast, easy and reliable manner the depletion of the antimicrobial agent(s) and/or the presence of (specific) pathogenic bacteria and/or agents which speed up healing of the tissue, minimize or prevent scarring.

A further aspect of the present invention relates to a method for providing controllable release of an antimicrobial agent comprising contacting the antimicrobial nanocapsules or the antimicrobial composition of the invention with an agent causing the permeation or destruction of the antimicrobial nanocapsules having incorporated at least one antimicrobial agent, wherein the agent causing the permeation or destruction of the nanocapsules is a membrane-permeating or membrane-lysing agent produced by pathogenic bacteria.

Preferably, the inventive method additionally comprises contacting signalling nanocapsules having at least one type of signalling agent encapsulated in a membrane with an agent causing the permeation or destruction of the signalling nanocapsules, wherein the agent causing the permeation or destruction of the nanocapsules is a membrane-permeating or membrane-lysing agent produced by pathogenic bacteria.

It is also preferred that the signalling agent(s) is/are released from the signalling nanocapsules after a predetermined time period after release of the antimicrobial agent(s).

A closely related aspect of the invention relates to a method, in particular an ex-vivo method for controlling pathogenic bacteria, comprising the steps:
(a) providing antimicrobial nanocapsules having at least one antimicrobial agent encapsulated in a membrane according to any one of claims 1-4 or providing the antimicrobial composition according to any one of claims 5-10 comprising such antimicrobial nanocapsules;
(b) contacting pathogenic bacteria which produce membrane-lysing or membrane-permeating agents with the nanocapsules of step a) causing lysis of said nanocapsules or the formation of pores in said nanocapsules and release of the antimicrobial agent(s) resulting in the killing of the pathogenic bacteria.

More specifically, in step (a) i) antimicrobial nanocapsules having at least one anti-microbial agent encapsulated in a membrane, ii) signalling nanocapsules having at least one type of signalling agent encapsulated in a membrane and, optionally, iii) wound healing nanocapsules having at least one wound healing agent encapsulated in a membrane, are provided, and the presence of the pathogenic bacteria causes release of the antimicrobial agent(s), the signalling agent(s) and the wound healing agent(s).

The antimicrobial nanocapsules and signalling nanocapsules and/or wound healing nanocapsules may also be combined in hybrid nanocapsules containing different types of active agents in the same or different compartments, in particular in hybrid nanocapsules as described above.

The invention is further illustrated by the following non-limiting Examples and Figures.

### FIGURES

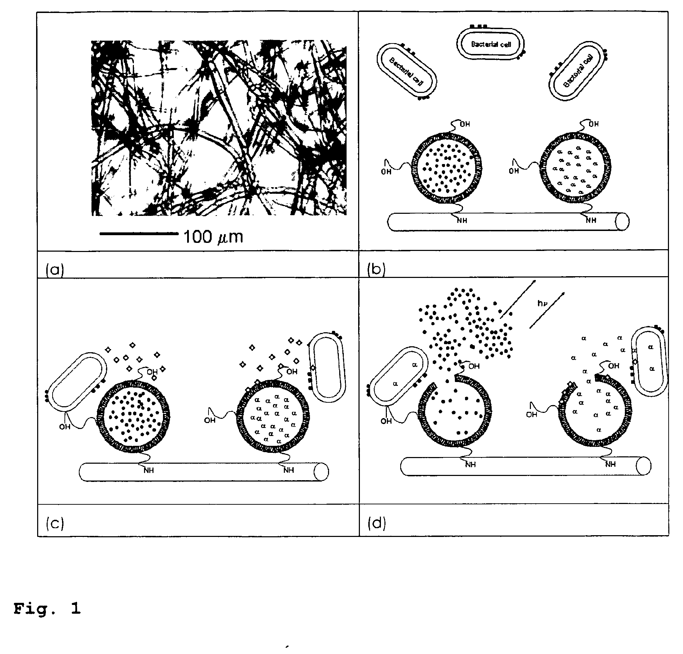

**Fig. 1****.** Schema of a responsive antimicrobial system of the invention
   **a)** Non-woven polypropylene textile material (light microscopy image) used in burns dressings. Plasma modified with reactive chemical groups. Nanocapsules sprayed on and bound to fibres.
   **b)** PEG stabilised nanocapsules containing 'switched off' dye on left and antibiotic/antimicrobial on right. PEG helps prevent non-specific attack from non-bacterial enzymes in wound exudate.
   **c)** Pathogenic bacteria release toxins and lipases which damage nanocapsules shell.
   **d)** Nanocapsules release signalling molecules that switch on (illumination), and antibiotic/antimicrobials.
**Fig. 2****.** Selective reduction of the pathogenic bacteria P. aeruginosa PA01 and S. aureus MSSA 476 in comparison with non-pathogenic E. coli DH5α by antimicrobial vesicles in suspension
**Fig. 3****.** ATR-FTIR data for the plasma deposition and subsequent attachment of vesicles on polypropylene non-woven
   **a)** FT-IR Polypropylene non-woven
   **b)** FT-IR of pulse plasma deposited maleic anhydride (MA) on non-woven polypropylene
   **c)** FT-IR of attached vesicles on MA modified polypropylene
**Fig. 4****.** Results from JIS measurement of the bacterial survivability on antimicrobial vesicle-modified fabric
   **a)** pathogenic bacteria P. aeruginosa PA01
   **b)** pathogenic bacteria S. aureus MSSA 476
   **c)** non-pathogenic E. coli DH5α
**Fig. 5****.** Fluorescence imaging of surface-attached vesicles exposed to the three species of bacteria from Fig. 4

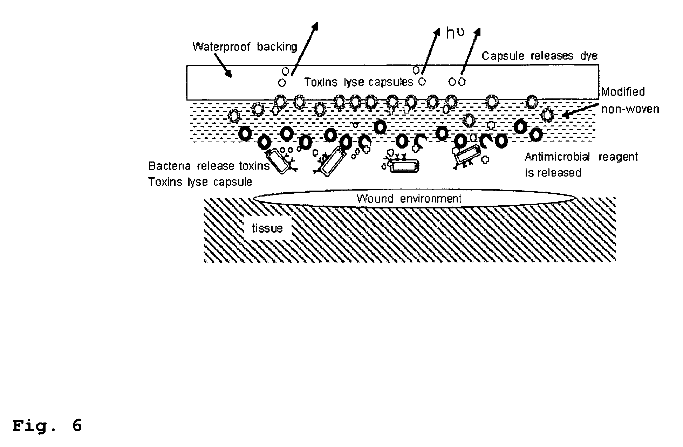

**Fig. 6****.** Schematic presentation of the composition and action of a wound dressing of the invention

### EXAMPLE 1

### Synthesis and Purification of Giant Unilamellar Vesicles (GUVs )

All chemical used in this work were obtained from Sigma-Aldrich, UK, and not purified further. The 1,2-Dimyristoyl-sn-Glycero-3-phosphocholine (DMPC) and 1,2-Dimyristoyl-sn-Glycero-3-phosphoethanolamine (DMPE) lipid were purchased from Avanti Polar Lipids, USA. GUVs were synthesized according to reference 70: The mixture of 0.041 g of DMPC and 0.002 g of DMPE with 0.013 g cholesterol was added to a vial with 1 mL chloroform (Fischer). A 200 µL aliquot of this solution was added to a 50 mL round-bottomed flask containing 980uL chloroform and 150 µL methanol (Fisher).

In a clean vessel, 100 mg sodium azide (15 mg mL^{-l}) or fluorescein (5 mM) was dissolved in 6.65 mL Tris buffer, pH 7.4. The stock azide or fluorescein mixture was then vortexed briefly and carefully added to the round bottomed flask containing the lipids. The resultant mixture was rotary-evaporated for 2 minutes at 40 °C. After the 2 minutes, the liquid in the round-bottom flask was observed to be a faint milky-white opalescent fluid which contained Giant Unilamellar Vesicles, GUVs. The GUVs were then purified by passing through NAP-10 columns to remove non-encapsulated sodium azide or fluorescein.

### EXAMPLE 2

### Determination of MIC, antimicrobial vesicle activity and fluorescein release

The minimum inhibitory concentration of sodium azide for the overnight cultures of the three bacterial strains: *Staphylococcus aureus* (MSSA 476), *Pseudomonas aeruginosa* (PA01), and E. *coli* DH5α grown in LB was determined using standard methods. For the vesicle lysis/release measurement, 100 µ1 of bacteria at a concentration of 10¹⁰ CFU mL⁻¹ was added to a suspension of 900 µ1 of vesicles. Every twenty minutes the bacteria concentration would be determined by standard plating assays and colony counting. Vesicles containing encapsulated fluorescein were immobilized on a polystyrene Petri dish *(vida-infra)* modified with a film of plasma deposited maleic anhydride. The GUVs were imaged using a Nikon eclipse TE2000-S epi-fluorescence microscope.

***MIC measurements** -* Initial measurements were made to determine the minimal inhibitory concentration (MIC) of sodium azide against the three test species of bacteria. It was important to determine the sensitivity of the test species to the antimicrobial agent, a this informs the analysis of later vesicle lysis measurements by the bacteria. If *E. coli* was much less susceptible to sodium azide than the other species, this could explain it's insensitivity to sodium azide containing vesicles. Fortunately, the E. *coli* proved to be the most susceptible to sodium azide (Table 1), making it an excellent non-pathogenic control strain.

**Table 1: MIC of sodium azide against P. aeruginosa, S. aureus and E. coli**

| **Bacteria Species** | **MIC** |
|---|---|
| *Pseudomonas aeruginosa* PA01 | 44 µg ml-¹ |
| *Staphylococcus aureus* MSSA 476 | 186 µg m1-¹ |
| *Escherichia coli* DHSα | 34 µg ml-¹ |

### Time resolved bacterial killing by vesicles in suspension

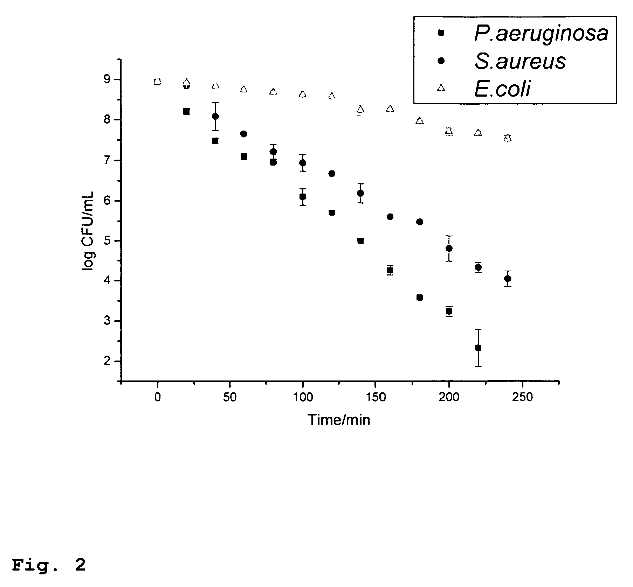

Measurements of the bacterial mediated vesicle lysis (and subsequent self-destruction) were made by sampling and plating out the viable bacterial population every 20 minutes. Results are shown in Fig. 2, with rapid loss of P. *aeruginosa,* which also has a low MIC against sodium azide; slightly lower loss of S. aureus (higher MIC for sodium azide), but very little loss of the most susceptible E. coli, despite this being the most sensitive to sodium azide. The conclusion from this experiment was that bacterial toxin mediated bacterial killing took place (Fig. 2).

### EXAMPLE 3

### Plasma deposition of maleic anhydride

The utility of plasma deposited maleic anhydride for attachment of proteins, DNA or other material has been detailed by the inventors in the literature (Jenkins A.T.A, Hu J, Wang Y.Z, Schiller, S, Foerch R, Knoll, W., Pulsed plasma deposited maleic anhydride thin films as supports for lipid bilayers, Langmuir,2000, 16, 6381-6384). This plasma deposition system uses a copper coil to couple rf power via an inductance matching system to an evacuated glass tube terminated with stainless steel glass flanges that are connected to earth. A home built pulse generator allowed for either continuous (CW) or pulsed plasma treatment. The modulation is typically described by the duty cycle (dc= tₒₙ / (tₒₙ + t_{off})) which is generally in the order of milliseconds. Maleic anhydride powder was freeze dried three times just prior to deposition to remove adsorbed water and then deposited using low power 1/40 at 50 W input power to afford the a high retention of anhydride and ring structure in the deposited film.

### Attachment of vesicles to pp-MA on non-woven polypropylene

The utility of vesicles as anti-infective agents in, for example, wound dressings requires that they be can be attached to fabric. For this part of the study, non-woven polypropylene (taken from the topsheet of commercially available disposable nappies), was coated in a plasma with a thin film of maleic anhydride under 1 / 40 ms / ms pulse conditions, to ensure a high degree of ring retention. The anhydride ring is sensitive to nucleophilic attack by primary amines (shown in the following scheme 1). The immobilization procedure is based on earlier work by Chifen, Förch, Jenkins et al.(Langmuir 2007).

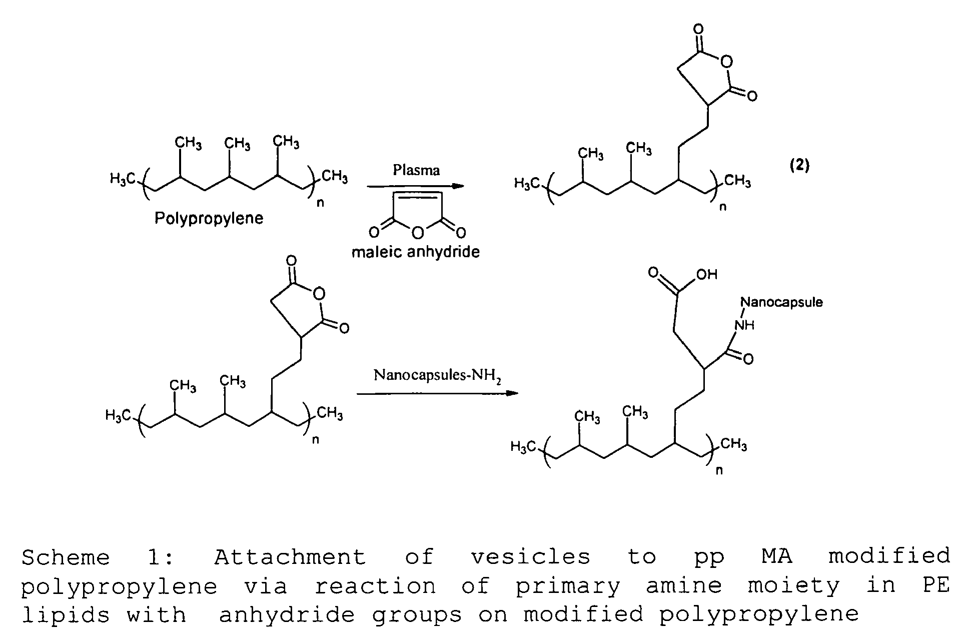

### EXAMPLE 4

### FITR analysis of attached vesicles

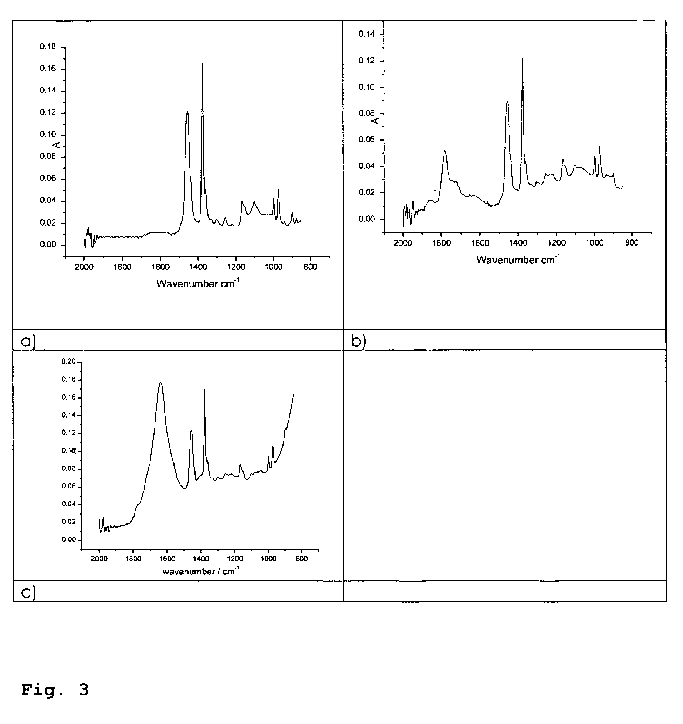

FTIR (Fig. 3) shows polypropylene non-woven; maleic anhydride ring retention following pp-MA deposition, and effect of adding vesicles to pp-MA modified non-woven. The polypropylene shows the expected C-H bend features. The ppMA modified polypropylene exhibits the expected anhydride group C=O absorption at 1780 and 1850 cm⁻¹ (as previously reported by various researchers *refs).* Attachment of the lipid vesicles to the pp-MA non-woven shows a strong, broad adsorption at 1630 cm⁻¹, resulting primarily from amide C=O in the reacted MA and from ester C=O in the fatty acid chains of the phospholipids.

### EXAMPLE 5

### Bacterial control by attached vesicles on pp-MA modified polypropylene

Having successfully immobilized vesicles on the pp-MA modified non-woven, the next part of the investigation looked at studying the antimicrobial performance of the modified fabrics against the two pathogenic bacteria (P. *aeruginosa* and S. *aureus)* and a non-pathogenic control, E. *coli.* The control gives important information on whether the fundamental hypothesis is correct: will pathogenic bacteria lyse the vesicles - and ensure their self-destruction, but non-pathogenic bacteria be largely unaffected?

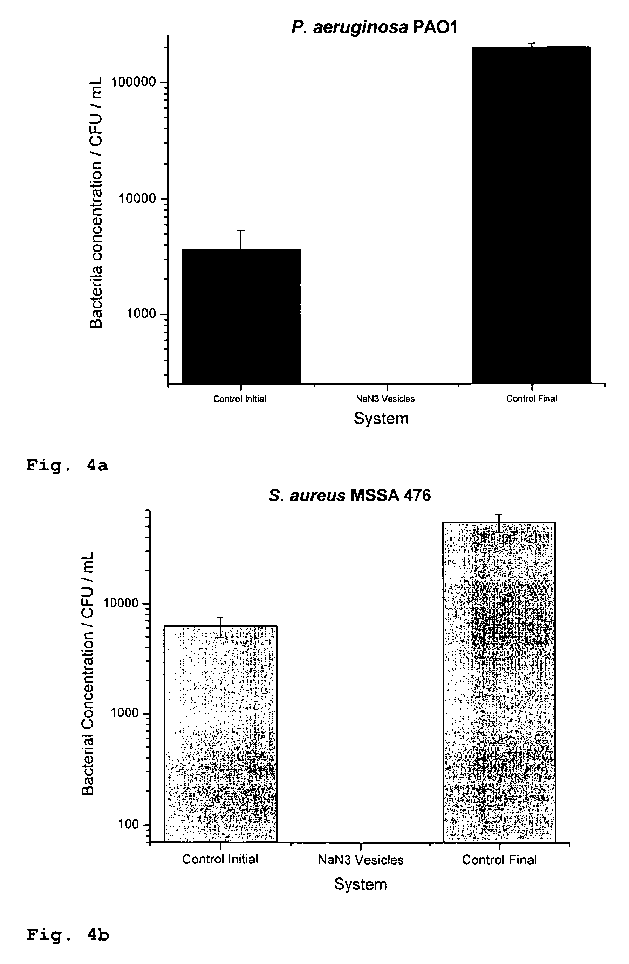

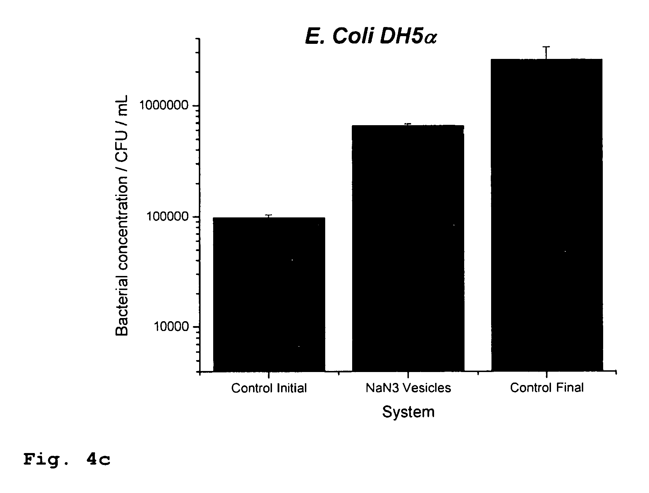

The antimicrobial performance of the sodium azide containing vesicles attached to fabrics was tested using a modification of the JIS 1902 standard method. The results in Fig. 4 show that the vesicle modified non-woven fabric completely kills both pathogenic species of bacteria *(P. aeruginosa* and S. *aureus),* whilst the non pathogenic *E. coli* (which cannot secrete toxins and lysing agents) grows at a rate only slightly less than the non-modified control. It is likely that some passive leakage of sodium azide controlled the growth of *E. Coli* to a small extent. The use of the E. coli control is further useful, as it allows a measure of the 'leakiness' of the vesicles containing sodium azide. This is being used in current and future research into improving the stability and reducing passive leakage from attached vesicles.

### EXAMPLE 6

### Fluorescence Microscopy of Vesicles attached to pp-MA modified Petri dishes

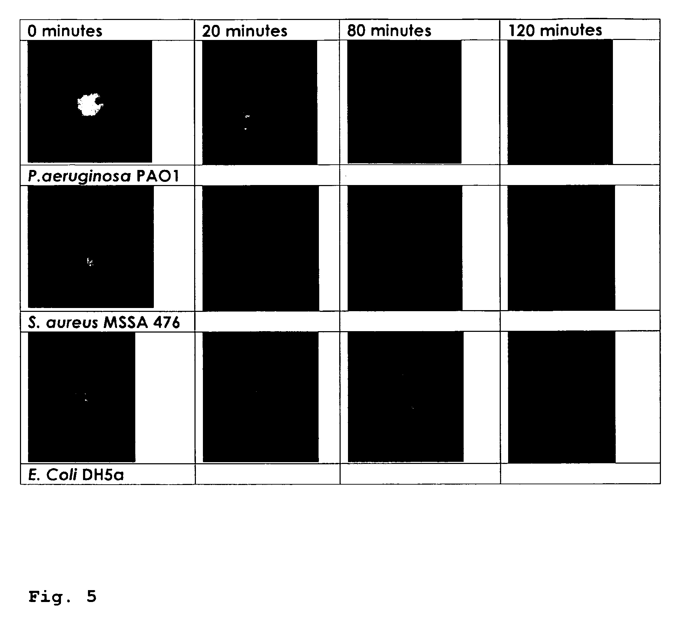

Vesicles containing fluorescein dye were imaged by fluorescence microscopy (Fig. 5). The images show that the vesicles undergo fairly rapid destruction following addition of the pathogenic S. *aureus* and P. *aeruginosa,* yet are unaffected by the addition of non-pathogenic *E. coli.* This further proves that the basic hypothesis is correct: vesicles can be made that are susceptible to pathogenic bacteria, but are unaffected by bacteria which do not secrete the enzymes/toxins that can lyse them.

## Claims

1. Antimicrobial nanocapsules having at least one antimicrobial agent encapsulated in a membrane, wherein the nanocapsules are capable to release the antimicrobial agent(s) in response to the presence of pathogenic bacteria which produce membrane-permeating or membrane-lysing agents.

2. The antimicrobial nanocapsules according to claim 1, wherein the nanocapsules are phospholipid-fatty acid vesicles, multicomponent polymerized acetylene fatty acid/synthetic lipid vesicles, amphiphilic block copolymer nanocapsules, nanocapsules comprising natural or synthetic polymers, protein capsules, or other containment systems comprising of self-assembled and/or polymerized organic molecules.

3. The antimicrobial nanocapsules according to claim 1 or 2, wherein the membrane-permeating or membrane-lysing agents are selected from the group of enzymes and other virulence factors and toxins, in particular lipases, phospholipases including phospholipase A2, toxic shock toxin (TSST-1), hyaluronidases, pore-forming toxins, in particular haemolysin, *S. aureus α,* β and δ toxins, streptolysin, and leukocidin.

4. The antimicrobial nanocapsules according to any one of claims 1-3, wherein the pathogenic bacteria are selected from the group of *Streptococcus,* in particular Group A and B *streptococci, Staphyloccous* species, *Klebsiella* species, *Pseudomonas* species, pathogenic *E. coli* species, *Lactobacillus, Helicobacter, Camphylobacter, Legionella, Listeria, Borellia, Yersinia, Bacillus* and *Vibrio* species.

5. An antimicrobial composition comprising the nanocapsules according to any one of claims 1-4.

6. The antimicrobial composition according to claim 5, which further comprises signalling nanocapsules having at least one type of signalling agent encapsulated in a membrane or shell, and/or wound healing nanocapsules having at least one wound healing agent encapsulated in a membrane or shell.

7. The antimicrobial composition according to claim 5 or 6, wherein the signalling nanocapsules and/or wound healing nanocapsules are also capable to release the respective agent(s) in response to the presence of pathogenic bacteria which produce membrane-permeating or membrane-lysing agents.

8. The antimicrobial composition according to claim 6 or 7, wherein the signalling nanocapsules and/or wound healing nanocapsules are adapted to release the respective agent(s) after a predetermined time period after release of the antimicrobial agent(s).

9. The antimicrobial composition according to any one of claims 6 to 8, wherein the antimicrobial nanocapsules and at least one of the signalling nanocapsules and the wound healing nanocapsules are combined in hybrid nanocapsules containing different types of active agents in the same or different compartments.

10. The antimicrobial composition according to any one of claims 5 to 9, which is provided on a substrate surface selected from the group of woven or non-woven fabrics, threads and yarns, solid polymeric surfaces, polymeric films, metal, glass or ceramic surfaces and/or contained within a hydrogel matrix such as chitosan.

11. A wound dressing comprising the antimicrobial nanocapsules or composition according to any one of claims 1-10.

12. A method for providing controllable release of an antimicrobial agent comprising contacting the antimicrobial nanocapsules or the antimicrobial composition according to any one of claims 1-10 with an agent causing the permeation or destruction of the antimicrobial nanocapsules having incorporated at least one antimicrobial agent, wherein the agent causing the permeation or destruction of the nanocapsules is a membrane-permeating or membrane-lysing agent produced by pathogenic bacteria.

13. The method according to claim 12, further comprising contacting signalling nanocapsules having at least one type of signalling agent encapsulated in a membrane with an agent causing the permeation or destruction of the signalling nanocapsules, wherein the agent causing the permeation or destruction of the nanocapsules is a membrane-permeating or membrane-lysing agent produced by pathogenic bacteria.

14. The method according to claim 13, wherein the signalling agent(s) is/are released from the signalling nanocapsules after a predetermined time period after release of the antimicrobial agent(s).

15. A method, in particular an ex-vivo method for controlling pathogenic bacteria, comprising the steps:
a) providing antimicrobial nanocapsules having at least one antimicrobial agent encapsulated in a membrane according to any one of claims 1-4 or providing the antimicrobial composition according to any one of claims 5-10 comprising such antimicrobial nanocapsules;
b) contacting pathogenic bacteria which produce membrane-lysing or membrane-permeating agents with the nanocapsules of step a) causing lysis of said nanocapsules or the formation of pores in said nanocapsules and release of the antimicrobial agent(s) resulting in the killing of the pathogenic bacteria.

16. The method according to claim 15, wherein in step (a) i) antimicrobial nanocapsules having at least one antimicrobial agent encapsulated in a membrane, ii) signalling nanocapsules having at least one type of signalling agent encapsulated in a membrane and, optionally, iii) wound healing nanocapsules having at least one wound healing agent, e.g. protease inhibitors or growth factors, encapsulated in a membrane, are provided, and wherein the presence of the pathogenic bacteria causes release of the antimicrobial agent(s), the signalling agent(s) and the wound healing agent(s).
